**Europäisches Patentamt**

(19) **European Patent Office**

**Office européen des brevets**

(11) Veröffentlichungsnummer: **0 058 987**
**B1**

(12) # EUROPÄISCHE PATENTSCHRIFT

(45) Veröffentlichungstag der Patentschrift:
**12.06.85**

(21) Anmeldenummer: **82101373.7**

(22) Anmeldetag: **24.02.82**

(51) Int. Cl.⁴: **C 07 C 43/23**, C 07 C 41/03,
C 07 C 41/46 // C07C41/42

(54) Verfahren zur Herstellung von Guajacolglycerinether.

(30) Priorität: **25.02.81 DE 3106995**

(43) Veröffentlichungstag der Anmeldung:
**01.09.82 Patentblatt 82/35**

(45) Bekanntmachung des Hinweises auf die Patenterteilung:
**12.06.85 Patentblatt 85/24**

(84) Benannte Vertragsstaaten:
**DE FR GB IT**

(56) Entgegenhaltungen:
**DE - A - 2 456 082**
**DE - A - 2 721 889**
**FR - A - 961 067**
**FR - A - 2 160 976**

(73) Patentinhaber: **Degussa Aktiengesellschaft,
Weissfrauenstrasse 9, D-6000 Frankfurt am Main 1 (DE)**

(72) Erfinder: **Merk, Wolfgang, Dr., Dipl.-Chem.,
Grünaustrasse 19, D-6450 Hanau 9 (DE)**
Erfinder: **Wagner, Rudolf M., Dr., Dipl.-Chem.,
Gutenbergstrasse 5, D-7918 Illertissen (DE)**
Erfinder: **Werle, Peter, Dr., Dipl.-Chem., Im Börner 43,
D-6460 Gelnhausen 2 (DE)**
Erfinder: **Nygren, Robert S., Dr., Dipl.-Chem.,
Fürstenbergstrasse 8, D-6450 Hanau 9 (DE)**

## Beschreibung

Guajacolglycerinether ist seit langem als Mittel für medikamentöse Lobotomie bekannt, s. Römpp, Chemie-Lexikon, 5. Auflage, Bd. II.

Er wird z.B. durch Umsetzung von Guajacol mit Epichlorhydrin in Gegenwart molarer Mengen Natriumhydroxid in Wasser hergestellt (Yakugaku Zasshi 87 [8], 967 [1967]). Diese Reaktion benötigt bei Temperaturen um 85 - 90°C jedoch relativ lange Reaktionszeiten und ergibt nur geringe Mengen (32%) an Guajacolglycerinether.

Ferner wird die Umsetzung mit Glycerin-α-monochlorhydrin beschrieben. Dabei ist es ebenfalls möglich, von Epichlorhydrin auszugehen und dieses in einer Vorstufenreaktion durch Zusatz einer geeigneten wässrigen Säure (z.B. 0,2% $H_2SO_4$) zum Glycerin-α-monochlorhydrin zu hydrolysieren (POL-PS 48 485 [1964]). Auch bei der Reaktion mit Glycerin-α-monochlorhydrin ist es notwendig, molare Mengen eines alkalischen Katalysators wie Natriumhydroxid anzuwenden. Die Reaktion wird hauptsächlich in Wasser, d.h. wässrigem Alkali oder wässrigem Methanol, durchgeführt (ES-PS 212 920 [1954], Czech-PS 107 107 [1963], POL-PS 42 379 [1959]). Allerdings ist auch der Einsatz von Toluol/Butanol-Mischungen beschrieben worden (DE-OS 2 256 506 [1973]).

Alle diese Verfahren verlaufen, wenn sie bei Raumtemperatur durchgeführt werden, nur sehr langsam, andererseits ist bei Temperaturen am Siedepunkt des Lösungsmittels mit beträchtlicher Farbstoffbildung zu rechnen. Darüber hinaus werden pro 100 kg Guajacol etwa 30 kg Natriumchlorid gebildet, die von der Reaktionslösung durch geeignete Massnahmen abgetrennt, zur Vermeidung von Ausbeuteverlusten gewaschen und entsorgt werden müssen. Weiterhin ist der Prozess durch einen beträchtlichen Energieaufwand gekennzeichnet, da Lösungsmittel mit hoher Verdampfungsenthalpie abzudestillieren sind.

Es ist ausserdem bekannt, 2,3-Epoxypropanol, d.h. Glycid, für die Umsetzung mit Phenolen, u.a. auch p-Methoxyphenol, heranzuziehen, s. BR-PS 628 497; als Katalysatoren fungieren tertiäre Amine oder quaternäre Ammoniumsalze.

Diese Reaktion mit Glycid anstelle von Glycerin-α-monochlorhydrin hat den grossen Vorteil, dass frei von Lösungsmitteln gearbeitet werden kann und kein Kochsalz anfällt. Dadurch fällt die Abtrennung des Kochsalzes fort, und das Produkt ist ausserdem frei von Chlor.

Der Nachteil dieser Reaktion liegt nach den Angaben der genannten britischen Patentschrift aber einmal in der nicht sehr hohen Ausbeute, die bei Einsetzen des genannten p-Methoxyphenols nur bei 50 bis 65% Rohprodukt liegt.

Ausserdem zeigte sich bei einer Übertragung dieser Reaktion auf o-Methoxyphenol, d.h. Guajacol, dass das erhaltene Produkt nur eine sehr geringe Reinheit aufweist. Es enthält noch einen beträchtlichen Teil des nicht umgesetzten Guajacols sowie polymere Begleitstoffe, ausserdem noch Reste der Katalysatoren. Auch ist das Produkt gefärbt.

Die Reinheit des Produktes lässt sich zwar durch Mehrfachdestillationen verbessern, aber abgesehen von dem technischen Aufwand, sinkt die Ausbeute dadurch natürlich noch weiter ab.

Auch nach dem Verfahren der DE-A-27 21 889, bei dem kernbromierte Phenole wie z.B. Bromphenol mit Glycid in Gegenwart von Alkalien mit oder ohne Anwesenheit eines Lösungsmittels umgesetzt werden, wurde eine ähnliche technische unbefriedigende Ausbeute und Reinheit erhalten.

Reaktionen mit Alkylenoxiden anstelle von Glycid — wie sie z.B. bei der Herstellung von Verjüngungsmitteln aus Phenolen und Alkylenoxiden nach der DE-A-24 56 082 in Gegenwart grösserer Mengen an Basen durchgeführt werden — lassen sich wesentlich einfacher als mit Glycid durchführen, da die Gefahr der Selbstpolymerisation aufgrund der fehlenden Hydroxylgruppe im Epoxidmolekül nicht besteht.

Aufgabe der Erfindung ist ein Verfahren zur Herstellung von Guajacolether, bei dem das Produkt in guter Ausbeute und Reinheit anfällt.

Es wurde nun gefunden, dass sich Guajacolglycerinether in sehr guter Ausbeute und Reinheit bei der Umsetzung von Glycid und einem Methoxyphenol ohne Anwesenheit eines Lösungsmittels, aber in Gegenwart eines Katalysators, bei Temperaturen von 80 bis 150°C herstellen lässt, wenn man o-Methoxyphenol mit Glycid in Gegenwart von einem Alkalihydroxid, einem Alkalialkoholat, einem Alkalicyanat und/oder Alkalithiocyanat in der Weise umsetzt, dass man Glycid in das geschmolzene Guajacol in dem Masse einführt, dass das eingeführte Glycid augenblicklich zu Guajacolglycerinether umgesetzt wird.

Die genannten Katalysatoren werden in Mengen von 0,0003 bis 0,1 Mol/Mol Guajacol verwendet. Bevorzugt sind Mengen von 0,003 bis 0,05 Mol/Mol Guajacol.

Als Alkalimetalle gelten Lithium, Natrium und Kalium. Besonders geeignete Katalysatoren sind Natriumhydroxid und/oder Kaliumcyanat. Die Katalysatoren werden am besten in ihrer handelsüblichen Form eingesetzt. Als Alkali-Alkoholate eignen sich vor allem Natrium- oder Kaliumethylat bzw. -äthylat.

Wenn die Katalysatoren in fester Form vorliegen, können sie in feingepulverter Form verwendet werden. Bevorzugte Reaktionstemperaturen liegen bei 90 bis 100°C.

Die Reaktanten können in äquivalenten Mengen eingesetzt werden, obwohl ein geringer Überschuss an Glycid, d.h. bis zu 10 Molprozent, vorzuziehen ist.

Das Rohprodukt, dessen Guajacolgehalt i.a. unter 0,5 Gewichtsprozent liegt, wird einer Destillation, am besten in einem Filmverdampfer, z.B. einem Dünnschichtverdampfer, unterworfen. Vor der Destillation werden die eingesetzten alkalischen Katalysatoren mit Säure inaktiviert. Die Art der Säure, ob anorganisch oder organisch, ist gleichgültig, ebenso die Säurestärke. Wichtig ist nur, dass die Säuren in dem für die Reaktion angegebenen Temperaturbereich stabil und nicht flüchtig oder zersetzlich sind. Sehr günstig ist die Verwendung niederer aliphatischer Carbonsäuren, wie Ameisensäure, Essigsäure, Propionsäure.

Die Säuren werden in äquivalenten Mengen, bezogen auf die alkalischen Katalysatoren, eingesetzt.

Der nach der Destillation anfallende Guajacolether ist farblos. Wenn gewünscht, kann er noch einer Umkristallisation in hierfür bekannten Lösungsmitteln, z.B. Estern niedermolekularer aliphatischer Carbonsäuren, wie z.B. Methyl- bis Propylacetat, oder Alkoholen wie Methanol oder Äthanol, unterworfen werden. Der Guajacolgehalt, der nach der Destillation etwa 0,3 Gew.-% betrug, ist nach dem Umkristallisieren auf praktisch Null reduziert.

Wasserdampfdestillationen haben sich zur Reinigung des Guajacolglycerinethers nicht bewährt.

Es wurde weiter gefunden, dass eine besonders wirksame Reinigung des Rohproduktes stattfindet, wenn in die nach der Reaktion vorliegende Schmelze aus Guajacolglycerinether — bevorzugt ohne jede Abkühlung — 0,05 bis 0,1 Mol Wasserstoffperoxid (100 gew.%ig) pro Mol eingesetztes Guajacol als wässrige Lösung eingeführt wird. Das Wasserstoffperoxid kann in handelsüblichen Konzentrationen, bevorzugt als 30 - 70 gew.%ige Lösung, verwendet werden.

Durch Zugabe des Wasserstoffperoxids werden anscheinend die farbgebenden Substanzen zerstört.

Die darauffolgende Kristallisation des Guajacolglycerinethers in Gegenwart des obengenannten Lösungsmittels (in an sich bekannter Weise) ist hierdurch erleichtert.

Bei Verwendung von z.B. Ethylacetat wird die Schmelze unter den Siedepunkt des Lösungsmittels auf etwa 70°C abgekühlt und darauf die ein- bis eineinhalbfache Gewichtsmenge an Ethylacetat, bezogen auf die Menge der Schmelze, hinzugefügt.

Der auskristallisierte Guajacolglycerinether ist praktisch frei von Guajacol.

Die Ausbeute kann durch Aufarbeiten der Mutterlauge noch weiter erhöht werden. Hierzu wird das Lösungsmittel abdestilliert, z.B. über einen Dünnschichtverdampfer, und aus dem Rückstand weiteres Rohprodukt gewonnen, das durch Destillation und Kristallisation — wie oben beschrieben — in Reinprodukt überführt werden kann.

Der Vorteil des erfindungsgemässen Verfahrens liegt in der stark erhöhten Ausbeute und der sehr grossen Reinheit des Produktes, die die Pharmakopoe-Spezifikationen bei weitem übertrifft. Der freie Guajacolgehalt des Reinproduktes liegt weit unter 0,03 Gew.-%, teilweise sogar unter 0,01 Gew.-%, s. Beispiel 5; dieser Gehalt wurde gaschromatographisch bestimmt. Ebenso ist die Farbzahl nach Hazen sehr gering.

Die Anmeldung wird an nachfolgenden Beispielen näher erläutert:

### Beispiel 1

In einem Kolben werden 248 g (2 Mol) Guajacol unter Rühren auf 90°C erhitzt, 0,01 Mol gepulvertes NaOH zugegeben und im Verlauf von 2 Stunden 151,7 g (2,05 Mol) Glycid zugetropft bei Konstanthalten der Temperatur. Die homogene, gelbliche Lösung wird weitere 2 Stunden bei der angegebenen Temperatur gehalten und mit 0,01 Mol Essigsäure behandelt. Die gaschromatographische Analyse ergibt 372 g (93,8% d.Th.) Guajacolglycerinether sowie einen Restgehalt von 0,4 Gew.-% an freiem Guajacol.

### Beispiel 2

Der Ansatz wird wie in Beispiel 1 beschrieben durchgeführt, als Katalysator werden jedoch 0,006 Mol gepulvertes KOCN eingesetzt. Ausbeute: 373 g (94,2% d.Th.) Guajacolglycerinether, sowie ein Restgehalt von 0,2 Gew.-% freies Guajacol.

### Beispiel 3

Der Ansatz wird wie in Beispiel 2 beschrieben durchgeführt, jedoch werden 155,4 g (2,10 Mol) Glycid zugegeben. Ausbeute: 186,3 g (94,1% d.Th.) Restgehalt an Guajacol 0,03 Gew.-%.

### Beispiel 4

### Aufarbeitung durch Destillation

Die Ansätze von 1 und 2 können wie folgt aufgearbeitet werden:
1000 g der warmen Reaktionsschmelze werden über einen Dünnschichtverdampfer bei 200°C / 1 mbar destilliert. Die Ausbeute an völlig farblosem Guajacolglycerinether beträgt 854 g (87% d.Th.).

### Beispiel 5

### Aufarbeitung durch Behandlung der Schmelze

Die nach der Reaktion vorliegende Schmelze nach Beispiel 1, 2 oder 3 wird wie folgt aufgearbeitet. Zur etwa 80°C warmen Schmelze werden 10 ml 30 Gew.-% $H_2O_2$ (0,09 Mol 100%iges $H_2O_2$) getropft und etwa 10 Min. gerührt. Nach Erreichen von 72 - 70°C werden anschliessend 500 ml Essigsäureäthylester zugegeben und im Verlauf von ca. 10 h kaltgerührt. Die ausfallenden Kristalle werden abgesaugt, gewaschen und getrocknet. Ausbeute: 324 g (82% d.Th.) Gehalt an freiem Guajacol < 0,01 Gew.-%.
Die Hazen-Farbzahl liegt bei etwa 10.

Aus den Mutterlaugen werden nach Abdestillieren des Essigsäureäthylesters — entweder durch längeres Stehenlassen oder durch Destillation — weitere Mengen an Guajacolglycerinether gewonnen. Die Ausbeute lässt sich dadurch um ca. 25 g auf etwa 88% d.Th. steigern.

### Patentansprüche

1. Verfahren zur Herstellung von Guajacolglycerinether durch Umsetzung von Methoxyphenolen mit Glycid bei Temperaturen von 80 bis 150°C, in Gegenwart von Katalysatoren, dadurch gekennzeichnet, dass man o-Methoxyphenol mit Glycid in Gegenwart von einem Alkalihydroxid, einem Alkalialkoholat, einem Alkalicyanat und/oder Alkalithiocyanat in der Weise umsetzt, dass man Glycid in das geschmolzene Guajacol in dem Masse einführt, dass das eingeführte Glycid augenblicklich zu Guajacolglycerinether umgesetzt wird.

2. Verfahren nach Anspruch 1, dadurch gekennzeichnet, dass man den Katalysator in Mengen von 0,0003 bis 0,1 Mol/Mol Guajacol, bevorzugt von 0,003 bis 0,05 Mol/Mol Guajacol, einsetzt.

3. Verfahren nach Anspruch 1 und 2, dadurch gekennzeichnet, dass man als Katalysator Natriumhydroxid in gepulverter Form verwendet.

4. Verfahren nach Anspruch 1 und 2, dadurch gekennzeichnet, dass man Kaliumcyanat in gepulverter Form einsetzt.

5. Verfahren nach Anspruch 1 bis 4, dadurch gekennzeichnet, dass man die nach der Reaktion anfallende Schmelze bei der am Ende der Reaktion vorliegenden Temperatur mit wässrigem Wasserstoffperoxid in Mengen von 0,05 bis 0,1 Mol (100 gewichtsprozentiges $H_2O_2$) pro Mol eingesetztes Guajacol versetzt und darauf den Guajacolglycerinether in bekannter Weise in Gegenwart eines organischen Lösungsmittels auskristallisiert.

6. Verfahren nach Anspruch 1 bis 5, dadurch gekennzeichnet, dass man 30 bis 70 gewichtsprozentiges Wasserstoffperoxid verwendet.

## Claims

1. A process for the production of guaiacol glycerol ethers by reacting methoxyphenols with glycidol at a temperature of from 80 to 150°C, in the presence of catalysts, characterised in that o-methoxyphenol is reacted with glycidol in the presence of an alkali metal hydroxide, an alkali metal alcoholate, an alkali metal cyanate and/or alkali metal thiocyanate in such a manner that glycidol is introduced into the molten guaiacol in a quantity, such that the glycidol introduced is reacted immediately to produce guaiacol glycerol ether.

2. A Process according to claim 1, characterised in that the catalyst is used in a quantity of from 0.0003 to 0.1 mol/mol of guaiacol, preferably from 0.003 to 0.05 mol/mol of guaiacol.

3. A process according to claims 1 and 2, characterised in that sodium hydroxide in powdered form is used as a catalyst.

4. A process according to claims 1 and 2, characterised in that potassium cyanate is used in powdered form.

5. A process according to claims 1 to 4, characterised in that the melts, resulting after the reaction, are treated at the temperature existing at the end of the reaction with aqueous hydrogen peroxide in a quantity of from 0.05 to 1.0 mol (100% by weight $H_2O_2$) per mol of guaiacol used, and the guaiacol glycerol ether is then crystallised in known manner in the presence of an organic solvent.

6. A process according to claims 1 to 5, characterised in that from 30 to 70% by weight of hydrogen peroxide is used.

## Revendications

1. Procédé de préparation de gaïacolglycérinéther par réaction de méthoxy-phénols avec le glycide à une température de 80 à 150°C, en présence de catalyseurs, procédé caractérisé en ce que l'on fait réagir l'o-méthoxy-phénol avec le glycide en présence d'un hydroxyde alcalin, d'un alcoolate alcalin, d'un cyanate alcalin et/ou d'un thiocyanate alcalin, en introduisant le glycide dans le gaïacol fondu à une allure telle que le glycide introduit se transforme instantanément en gaïacolglycinéther.

2. Procédé selon la revendication 1, caractérisé en ce que le catalyseur est mis en oeuvre dans une proportion de 0,0003 à 0,1 mole par mole de gaïacol, de préférence 0,003 à 0,05 mole/mole de gaïacol.

3. Procédé selon les revendications 1 et 2, caractérisé en ce que comme catalyseur on utilise l'hydroxyde de sodium sous forme pulvérulente.

4. Procédé selon les revendications 1 et 2, caractérisé en ce que le cyanate de potassium est utilisé sous forme pulvérulente.

5. Procédé selon les revendications 1 à 4, caractérisé en ce que dans la masse fondue obtenue après la réaction, à la température régnant à la fin de la réaction, on introduit de l'eau oxygénée en solution aqueuse dans une proportion de 0,05 à 0,1 mole ($H_2O_2$ à 100% de poids) par mole de gaïacol mise en oeuvre et ensuite, on fait cristalliser le gaïacolglycérinéther de manière connue, en présence d'un solvant organique.

6. Procédé selon les revendications 1 à 5, caractérisé en ce que de l'eau oxygénée à 30-70% en poids est utilisée.